# EUROPEAN PATENT APPLICATION

(11) **EP 4 197 348 A1**
(43) Date of publication of application: **21.06.2023**
(21) Application number: 21214492.7
(22) Date of filing: 14.12.2021
(51) Int. Cl.: A23L 33/00, A61K 36/185, A61K 36/45, A61K 36/73, A61K 36/87, A61P 25/28

(54) **COMPOSITION AND METHOD OF IMPROVING COGNITION**

(71) Applicant: Berry Lab AB, 220 07 Lund (SE)
(72) Inventor: HEYMAN-LINDÉN, LOVISA, 222 23 LUND (SE); MARUNGRUANG, Nittaya, 224 79 LUND (SE); HUANG, Fang, 222 40 LUND (SE); ÖSTE, Rickard, 226 49 LUND (SE)
(74) Representative: Brann AB

(57) **Abstract**

The invention provides a composition comprising disintegrated bilberry (*Vaccinium myrtillus*) berries and disintegrated lingonberry (*Vaccinium vitis-idaea*) berries. A method of manufacturing the composition, as well as a method of amelioration of a cognitive function, such as cognition, memory, and working memory, in a healthy human subject, are also provided.

## Description

### FIELD OF THE INVENTION

The present invention relates to the field of compositions and methods of improving cognition, including memory, in healthy subjects using such compositions.

### BACKGROUND OF THE INVENTION

Humankind has throughout history sought means and methods for improving cognition and memory. Such improvements would inter alia allow humans to carry out complex tasks faster and easier, as well as to solve more complex problems. In addition, a sense of improved cognition or memory, i.e., a sense of being focused, provides a pleasant feeling to the individual. Additionally, as humans age, cognition naturally decreases, thus providing a further desire to maintain the cognitive functions.

Accordingly, various compounds have been used to provide improvement of memory and cognition. Caffeine, for example, is widely used in the form of the beverage coffee to provide a stimulating effect of the central nervous system to thereby reduce fatigue and drowsiness and improve alertness. The effects further extend to learning and memory and improving reaction time and concentration. Another compound, nicotine, is also widely used in the form of tobacco to provide cognitive-enhancing effects on attention, working memory, fine motor skills and episodic memory function. Other compounds such as Modafinil and donepezil have been shown to improve helicopter and airline pilot's simulator performance.

The above exemplified and similar compounds however have various drawbacks including inter alia dependency, the association with other adverse health effects, and in the case of the more potent compounds, the high risk of drug abuse which mandates a need for restriction/prescription, and which in turn may result in a market for procuring and distributing these compounds illegally.

As an alternative, benefits from eating certain diets and foods have gained interest within the area of brain research.

It is thus an object to the present invention to provide a composition for improving cognition.

It is a further object of the present invention to provide a method of manufacturing such a composition.

It is a yet further object of the present invention to provide a method of ameliorating a cognitive function in healthy human subjects.

### SUMMARY OF THE INVENTION AND DETAILED DESCRIPTION

At least one of the above objects, or at least one of the objects which will be evident from the below description, is according to a first aspect of the invention achieved by a composition comprising disintegrated bilberry *(Vaccinium myrtillus)* berries and disintegrated lingonberry *(Vaccinium vitis-idaea)* berries.

The present invention is thus based on the finding by the present inventors that bilberry and lingonberry berries, when administered in disintegrated form to mice, provides significant improvements to cognition and memory in mice, as shown in Example 1. Specifically, as described in further detail in said example, both young and old healthy mice fed that berry composition obtained improved performance in a T-maze task, and also obtained improved performance in Barnes maze, thus showing that these mice had improved spatial working memory, active working short term memory, as well as improved spatial memory, working memory, memory, and cognition.

The composition is preferably a drinkable composition. The term drinkable encompasses that the composition is liquid or at least semi-liquid so that it can be consumed similar to how a beverage such as water is consumed. In other words, the liquid composition is not a solid composition. A drinkable composition thus encompasses a liquid or semiliquid composition, more preferably the drinkable composition is a liquid composition. The drinkable composition may be in the form of beverage, a viscous composition such as a smoothie or soup or drink with similar viscosity. A serving of the composition preferably has a volume of from 50-500 ml, such as from 50-400 ml, preferably from 50-300 ml, such as from 100-400 ml, preferably from 100-300 ml. Typically 1-3 servings of the composition may be consumed per day (24h) for an adult human. The composition may further comprise additives such as sweeteners, flavourings, preservatives, colouring agents. Each serving of the composition may be packaged in its own package to render it ready for consumption, or alternatively several servings of the composition may be packaged in a package for pouring out individual servings as needed. The composition may be consumed on its own, e.g., between meals, or alternatively as a meal companion, i.e., together with a meal.

The composition may alternatively be in the form of a powder composition. Such a powder composition may be obtained by drying, preferably freeze-drying, the disintegrated bilberry and lingonberry berries. More preferably a powder composition may be obtained by freeze-drying the drinkable composition. The powder composition may be consumed as is or mixed with food. Further, the powder composition may be mixed with water or other liquid to form a drinkable composition.

Accordingly, disintegrated berry encompasses dried, e.g., freeze-dried, disintegrated berry.

The powder composition may be provided in servings containing an amount of disintegrated bilberry berries and disintegrated lingonberry berries corresponding to the amounts in the servings of the drinkable composition.

The berries are disintegrated. This means that the berries have been divided, such as e.g., by crushing, cutting, mincing, milling, homogenization, into smaller particles. The degree of disintegration may be adjusted to modify the mouthfeel of the composition, where, generally, a high degree of disintegration and corresponding small size of the berry particles provides a composition that has a smooth mouthfeel.

Homogenization used herein encompasses both disintegrating by forcing the material to be homogenized through a nozzle at high pressure so as to cause the material to experience high shear, turbulence and/or cavitation so as to cause the material to break into smaller particles, as well as milling the material to be homogenized in a mill, in particular in a colloid mill such as a toothed colloid mill.

The berries may further be disintegrated after being frozen and/or dried.

The composition preferably comprises disintegrated whole bilberry and lingonberry berries, i.e., the composition preferably comprises disintegrated whole bilberry berries and disintegrated whole lingonberry berries. This means that all parts of the berries, following disintegration, are found in the composition. In other words, the composition is not made up only of certain fractions or parts of the whole berries, nor are the disintegrated whole berries filtered or otherwise treated to remove some constituent of the whole berries prior to forming the composition. Preferably only the whole berries, and no other parts, such as leaves of the respective plants, are present in the composition.

It is further contemplated within the context of the present invention that one of bilberry berries and lingonberry berries may be present in the composition in the form of disintegrated whole berries, whereas the other is present in to form of an extract, concentrate, filtrate, or fraction of the whole berry.

Preferably the composition comprises at least 5 wt%, such as 5 wt% to 73 wt%, preferably 5 wt% to 67 wt%, more preferably 5 wt% to 50wt%, such as 10 wt% to 40 wt%, such as 18.25 wt% to 36.5 wt%, in total of disintegrated bilberry berries and disintegrated lingonberry berries.

The wt% relates to the weight of the berries prior to disintegration and includes any liquids released during the disintegration of the berries. Accordingly, 100 g composition preferably contain disintegrated berries obtained from an amount of bilberry berries and lingonberry berries together weighing at least 5 g prior to disintegration of the berries. In addition to the above given wt%, the composition may comprise liquid such as water, juice from fruits or berries other than bilberry and lingonberry, additives as described above, added fibers, proteins, fats, etc.

Alternatively, at least 67 wt% of the composition is made up of disintegrated bilberry berries and disintegrated lingonberry berries.

Further alternatively, at least 75 wt% of the composition may be made up of disintegrated bilberry berries and disintegrated lingonberry berries.

In one embodiment at least 35 wt% of the composition is made up of disintegrated bilberry berries, and at least 35 wt% of the composition is made up of disintegrated lingonberry berries, resulting in a total of 70 wt%.

Preferably the composition comprises equal wt% of disintegrated bilberry berries and disintegrated lingonberry berries.

This provides good effects on cognition from both berries.

Preferably the bilberry berries and lingonberry berries are disintegrated, such as by homogenization, such that 90% of the number of particles of the disintegrated berries have a diameter less than 10 µm, preferably less than 5 µm, more preferably 4.2 µm or less.

This is advantageous in that it has been found to provide a good texture to the composition. Further, by disintegrating the berries to these particle sizes and distributions, compounds in the berries responsible for the cognition enhancing effects become released from the structures in the berries and may also become more bioavailable to the consumer of the composition. Preferably homogenization is used to achieve these particle sizes and distribution.

Homogenization is preferable when disintegrating berries, especially whole berries, because all parts of the berries, i.e., peel, skin, seeds, which contain high levels of potentially bioactive compounds can be included in the composition without detrimental effect on taste and palatability.

Typically, 50% of the number of particles in the composition have a diameter of less than 5 µm, preferably less than 3 µm, more preferably 2.3 µm or less.

Typically, 10% of the number of particles in the composition have a diameter of less than 3 µm, preferably less than 2 µm, more preferably 1.8 µm or less.

Preferably the bilberry berries and lingonberry berries are disintegrated, such as by homogenization, such that 90% of the number of particles in the composition have a diameter of less than 4-6 µm, such as less than 4.2 µm, 50% of the number of particles in the composition have a diameter of less than 2-3 µm, such as less than 2.3 µm, and 10% of the number of particles in the composition have a diameter of less than 1-2 µm, such as less than 1.8 µm.

The (number) volume weighted mean diameter of the particles (D[4,3]-value) is preferably 75-125 µm, preferably 90-110 µm, more preferably 97 µm.

Alternatively, the bilberry berries and lingonberry berries are disintegrated, such as by homogenization, such that 90% of the volume of particles in the composition have a diameter less than 1000 µm, preferably less than 750 µm, more preferably 550 µm or less.

Typically, 50% of the volume of particles in the composition have a diameter less than 500 µm, preferably less than 250 µm, more preferably 165 µm or less.

Typically, 10% of the volume of particles in the composition have a diameter less than 100 µm, preferably less than 75 µm, more preferably 55 µm or less.

Preferably the bilberry berries and lingonberry berries are disintegrated, such as by homogenization, such that 90% of the volume of particles in the composition have a diameter of less than 500-600 µm, such as less than 550 µm, 50% of the volume of particles in the composition have a diameter of less than 100-200 µm, such as less than 165 µm, and 10% of the volume of particles in the composition have a diameter of less than 40-60 µm, such as less than 55 µm.

The (volume) volume weighted mean diameter of the particles (D[4,3]-value) is preferably 200-300 µm, preferably 220-260 µm, more preferably 240 µm.

Preferably the composition comprises one or more of disintegrated cloudberry (*Rubus chamaemorus*) berries, disintegrated sea buckthorn (*Hippophaë rhamnoides*) berries, and disintegrated blackcurrant (*Ribes nigrum*) berries. These additional berries are preferably included in the composition as disintegrated whole berries.

As shown in example 1, also these additional berries improve cognition. The inclusion of these types of disintegrated berries may advantageously be used to adjust taste and colour of the composition while retaining a high proportion of disintegrated berries in the composition so as to maintain an efficient cognition improving effect. The wt% of these additional disintegrated berries in the composition is preferably less than the wt % of either of the disintegrated bilberry berries and lingonberry berries, respectively. The sum wt% of these additional disintegrated berries may for example be from 1 wt% to 20 wt%.

Preferably the composition further comprises grape *(Vitis)* juice.

This is advantageous in that the grape juice decreases the viscosity of the composition, and further sweetens the taste, and/or reduces sourness, of the composition.

The grape juice is preferably obtained by juicing, e.g., pressing, grapes. The grape juice is more preferably filtered after pressing to remove any solid particles, e.g., skin particles or seed particles, from the pressed grapes.

In addition, or as an alternative, other fruit juices such as e.g., apple juice and orange juice may be included in the composition.

Preferably the composition is made up essentially of disintegrated bilberry berries, disintegrated lingonberry berries, and grape juice.

Such a composition has a pleasant taste and contains high amounts of disintegrated bilberry and lingonberry berries, and hence provide a good cognitive enhancing effect. Typically, the composition comprises 2-37 wt%, such as 5 to 20 wt%, such as 10 to 20 wt% of each of disintegrated bilberry berries and disintegrated lingonberry berries, and 2 to 40 wt%, such as 5 to 35 wt%, such as 10-30 wt%, more preferably 10-20 wt% of grape juice. The term made up essentially of encompasses consisting essentially of and means that only those further components which do not materially affect the effect of the disintegrated bilberry berries, disintegrated lingonberry berries, and grape juice, may be included. Additional components such as flavouring agents, aromas, colours, preservatives, sweeteners, viscosity modifying agents, additional water, vitamins, and minerals are considered components which do not materially affect the function or the effect of the composition.

Alternatively, the composition consists of disintegrated bilberry berries, disintegrated lingonberry berries, grape juice, and optionally water.

In one specific embodiment the proportions of each of disintegrated bilberry berries, disintegrated lingonberry berries, and grape juice, in relation to the combined amount of these three ingredients, is 36.5 wt%, 36.5 wt%, and 27 wt%, respectively.

More preferably the disintegrated bilberry berries and disintegrated lingonberry berries consist of disintegrated whole bilberry berries and disintegrated whole lingonberry berries.

At least one of the above objects, or at least one of the objects which will be evident from the below description, is according to a second aspect of the invention achieved by a method of manufacturing a composition, comprising the step of
i. providing a mixture of disintegrated bilberry berries and disintegrated lingonberry berries.

The composition is preferably a composition according to the first aspect of the present invention, hence the description of the composition according to the first aspect of the present invention above also applies to the method of manufacturing the composition according to the second aspect of the present invention. The disintegrated bilberry berries and disintegrated lingonberry berries are preferably disintegrated whole bilberry berries and disintegrated whole lingonberry berries.

The disintegrated bilberry berries and disintegrated lingonberry berries are preferably obtained by homogenizing each variety of berries separate, or by homogenizing a mixture of the berries. Preferably the berries are homogenized to the particle sizes and distributions described above.

The method may further comprise adding a liquid such as water to the bilberries and lingonberries prior to disintegrating the bilberries and lingonberries, and/or adding the liquid such as water to the mixture of disintegrated bilberry berries and disintegrated lingonberry berries so as to dilute the mixture.

The method may further comprise the step of:
ii. mixing the composition with one or more of disintegrated cloudberry berries, disintegrated sea buckthorn berries and disintegrated blackcurrant berries.

As described above, these other berries also improve cognition. The disintegrated cloudberry berries, disintegrated sea buckthorn berries and disintegrated blackcurrant berries, may be obtained by homogenization as described for the bilberry and lingonberry berries above. The size of the of the particles of the disintegrated cloudberry berries, disintegrated sea buckthorn berries and disintegrated blackcurrant berries may preferably be as for the disintegrated bilberry berries and the disintegrated lingonberry berries.

The disintegrated cloudberry berries, disintegrated sea buckthorn berries and disintegrated blackcurrant berries are preferably disintegrated whole cloudberry berries, disintegrated whole sea buckthorn berries, and disintegrated whole blackcurrant berries.

Preferably the method further comprises the step of:
iii. mixing the composition with grape juice.

As described above, grape juice provides sweetness and/or a lessening of sourness of the composition. The grape juice may advantageously be mixed with the berries prior to the disintegration of the berries as this may make the berries easier to handle during the disintegration

Preferably homogenization is used for disintegrating.

As mentioned above homogenization is preferably used such that 90% of the number of particles of the disintegrated berries have a diameter less than 10 µm, preferably less than 5 µm, more preferably 4.2 µm or less.

At least one of the above objects, or at least one of the objects which will be evident from the below description, is according to a third aspect of the invention achieved by a method of amelioration of a cognitive function, such as cognition, memory, and working memory, in a healthy human subject, the method comprising administering a composition according to the first aspect of the present invention, to the healthy human subject.

As seen in the example section, the composition is capable of ameliorating cognitive functions in mammals, e.g., humans.

The method is a non-therapeutic method as it is performed on healthy humans. Amelioration of a cognitive function encompasses improving a cognitive function, such as improving cognition, memory, and/or working memory.

The human subject is a healthy human subject. The term "healthy" has its general meaning, and here in particular refers to a subject who does not notably suffer from any illnesses or health complaints that influences or impairs a cognitive function. Accordingly, the subject whose cognitive function is ameliorated or improved by the method of the present invention has not been diagnosed with a cognitive impairment or memory disorder, and in particular has no cognitive impairment or memory disorder. The composition may be administered one or more times per day. The composition may for example be administered together with a meal such as breakfast, lunch, or dinner. Alternatively, the composition may be administered between meals such as a snack or following exercise.

Preferably the cognitive function is selected from the group consisting of spatial working memory, active working short term memory, spatial memory, working memory, memory, and cognition.

These types of cognitive functions are tested by the examples.

Preferably the composition is administered at a dose corresponding to at least 50 g, preferably at least 60 g more preferably at least 100g, of disintegrated bilberry and lingonberry berries per day.

As shown in the following examples and therein described figures, the consumption of the composition provides improved cognition to subjects.

An alternative first aspect of the present invention concerns a composition comprising one or more of disintegrated cloudberry *(Rubus chamaemorus)* berries, disintegrated sea buckthorn (*Hippophaë rhamnoides)* berries and disintegrated blackcurrant (*Ribes nigrum)* berries. The composition may be obtained and formulated as described above for the composition according to the first aspect of the present invention. The disintegrated cloudberry berries, disintegrated sea buckthorn berries and disintegrated blackcurrant berries are preferably disintegrated whole cloudberry berries, disintegrated whole sea buckthorn berries, and disintegrated whole blackcurrant berries.

The composition alternative first aspect of the present invention may be obtained similarly to the method according to the second aspect of the present invention by a method according to an alternative second aspect of the present invention.

An alternative third aspect of the present invention concerns a method of amelioration of a cognitive function, such as cognition, memory, and working memory, in a healthy human subject, the method comprising administering one or more of disintegrated cloudberry *(Rubus chamaemorus)* berries, disintegrated sea buckthorn (*Hippophaë rhamnoides)* berries, and disintegrated blackcurrant *(Ribes nigrum)* berries to the healthy human subject. The one or more disintegrated berries are preferably administered as the composition according to the alternative first aspect of the present invention, and are preferably disintegrated whole berries

A further alternative aspect of the present invention concerns a fraction of the composition according to the first aspect of the present invention. The fraction may for example be obtained by filtering or otherwise dividing the composition into different such fractions.

A further alternative third aspect of the present invention concerns a method of increasing the microbial abundance of *Akkermansia*, in a healthy human subject, the method comprising administering a composition according to the first aspect of the present invention to the healthy human subject.

### BRIEF DESCRIPTION OF THE FIGURES

Fig. 1A-1C show T-maze test results expressed as first entries into correct goal arm %
(Fig. 1A) frequency in correct goal arm % (Fig. 1B) and duration in correct goal arm %
(Fig. 1C), for young mice having consumed a composition according to the first aspect of the present invention.
Fig. 2A-2C show T-maze test results expressed as first entries into correct goal arm %

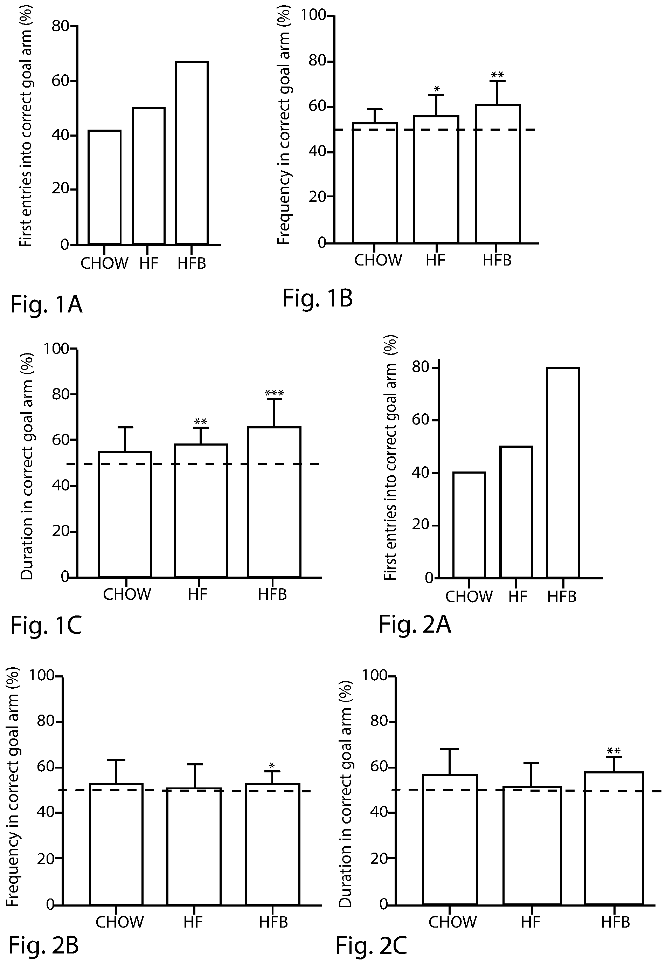

(Fig. 2A) frequency in correct goal arm % (Fig. 2B) and duration in correct goal arm %
(Fig. 2C) for adult/old mice having consumed a composition according to the first aspect of the present invention.

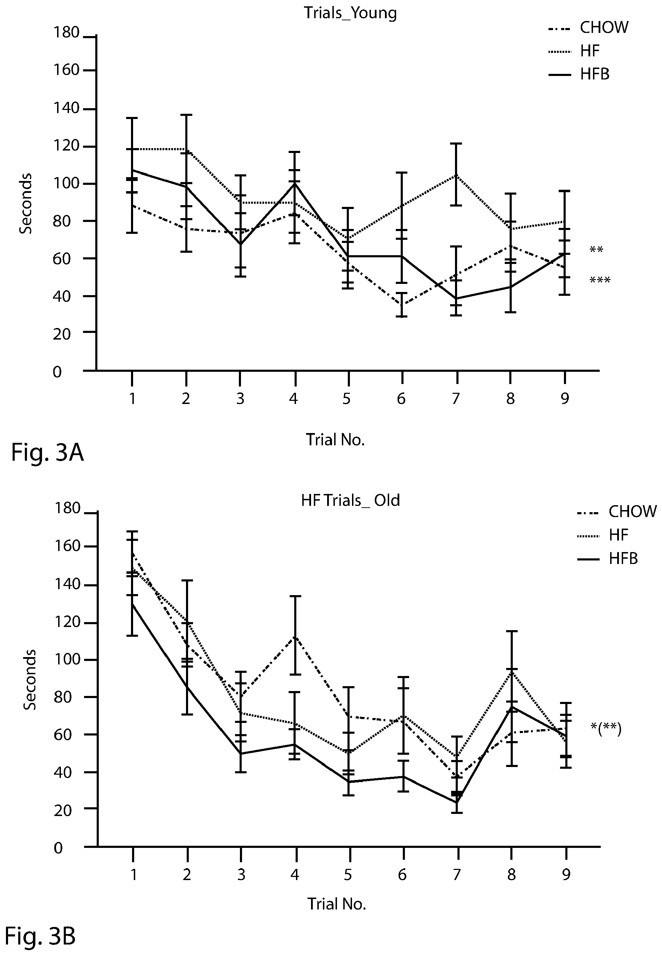

Fig. 3A shows Barnes maze test results for young mice having consumed a composition according to the first aspect of the present invention.
Fig. 3B shows Barnes maze test results for adult/old mice having consumed a composition according to the first aspect of the present invention.

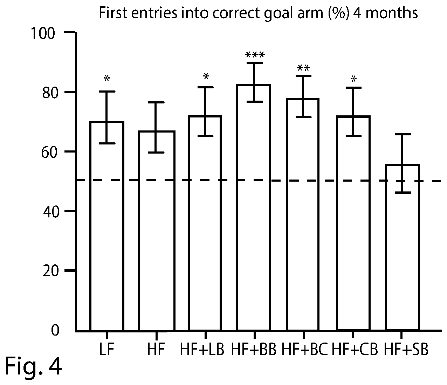

Fig 4 shows T-maze test results expressed as first entries into correct goal arm % for mice having consumed various diets including different Nordic berries.

### EXAMPLES

### Example 1 - A composition comprising disintegrated bilberry and Lingonberry improves memory and cognition in mice

C57BI/6J mice from Janvier lab (France) were used. Young (5 weeks at arrival) and aged (14 months at arrival) mice were included in the study. The individuals in the young group were divided in three diet groups, each consisting of 12 individuals. The food fed to the individuals in the three diet groups were standard chow, high fat (HF) (60 kcal% high-fat diet) respectively high fat with berries (HFB) (HF + 20 wt% of a composition according to the first aspect of the present invention, specifically made up of 36.5 wt% disintegrated whole bilberry berries, 36.5% disintegrated whole lingonberry berries, and 27 wt% grape juice, provided as freeze dried powder). The individuals in the aged group were divided into three diet groups, each consisting of 10-11 individuals. They were fed the same diets as young animals: standard chow, HF and HFB.

The feeding duration was 18 weeks.

The behaviour tests used were T-maze and Barnes maze. Monitoring was performed by a camera system using the Ethovision XT 14.0 (Noldus Information Technology B.V., Wageningen, The Netherlands) software for both video tracking and video analysis.

A modified T-maze tests was carried out as follows:

The T-maze was made of transparent acrylic and consisted of three arms (two goal arms: 30 cm x10 cm; one starting arm: 30 x 10 cm).

First, each mouse was allowed to explore the T-maze for 5 min with one of the goal arms blocked off.

Then, after a waiting time of 3-4 hours, the mouse was again allowed to explore the T-maze for 5 min, now having access to all three arms.

Based on the natural tendency of rodents to prefer exploring a novel environment over a familiar one, a mouse with a memory of the previously visited goal arm is expected to prefer visiting the previously blocked goal arm (i.e., correct goal arm) during the trial where no arms are blocked. The position of the mouse was recorded using video tracking software, allowing the later determination of the parameters:
First entries into correct goal arm % = proportion of mice per group that, when allowed to freely explore the maze, chose to make a first turn into the previously unexplored goal arm.

Frequency in correct goal arm % = number of visits to the previously unexplored arm in relation to total number of visits to both goal arms.

Duration in correct goal arm % = time spent in the previously unexplored arm in relation to total time spent in both goal arms.

Given that there may be a 50% chance that the mice make the visits (i.e., first entries, frequency, and duration) into the correct goal arm, mice showing the measured parameters deviating from the 50% chance indicate their ability to memorize the arm that has been visited in the previous trial. Hence this test is a measure of spatial learning and memory.

In the Barnes maze, mice are placed in the middle of a 100-cm diameter round table with 20 holes (5 cm diameter) around the edge, at height of 0,6 m from the floor. In one of the holes mice can escape into a box that is inserted under the table. The mice seek to avoid the brightly illuminated open space represented by the table, and therefore tries to find the hole leading to the escape box. The time required for the mice to find the box is measured. Each mouse is tested several times. When a mouse directly moves to the correct hole, without first exploring holes without the escape box, then this indicates that a spatial memory has formed. Typically, mice tested twice a day will learn the position of the correct hole within 5 days.

The results discussed below showed that individuals that were fed with the berries achieved improved cognition in both young and old groups.
Fig. 1A shows first entries into correct goal arm % in the young group during a T-maze test. As seen, mice receiving the HFB diet exhibited a higher than 50% chance on the parameter of first entries into the correct goal arm %. This was not seen in the groups receiving HF without berry supplementation, indicating that HFB diet improved spatial memory of the mice.
Fig. 1B and Fig. 1C show frequency in the correct goal arm % and duration in the correct goal arm % in the young group during a T-maze test. As seen, the mice receiving the HFB diet made more visits and spent more time in the correct arm of the maze, thus further indicating that these mice remembered and noted that they had not previously explored the correct goal arm.
Fig. 2A shows first entries into correct goal arm % in the aged group during a T-maze test. The data show that the first entries into correct goal arm % was higher for the mice in the HFB diet group.
Fig. 2B and Fig. 2C show frequency in the correct goal arm % and duration in the correct goal arm % in the aged group during the T-maze test.

As seen both the duration% and the frequency% were higher for the mice in the HFB diet group.

Fig. 3A and Fig. 3B illustrate results of the Barnes maze test, showing latency, i.e. the time required to find and enter the target hole, in the young and aged group, respectively. The figures illustrate that in both groups the individuals in the HFB diet groups showed a lower latency, i.e. mice receiving the berry composition learn to find the target hole faster compared to the control group. The effect is even more pronounced in the aged group.
Two-way ANOVA: HF vs CHOW***, HF vs HFB** in Fig. 3A.
Two-way ANOVA: HF vs HFB *, One-way ANOVA: HF vs HFB ** in Fig. 3B ***P < 0.001, **P < 0.01, *P<0.05

### Example 2 - Further effects of a composition comprising Bilberry and Lingonberry

The mice used in example 1 were further tested for the effects on gut microbiota. It was noted that the composition provided a statistically significant expansion of *Akkermansia* (increase in the relative abundances of the genus *Akkermansia* as well as its species level) in aged as well as young mice.

### Example 3 - Manufacturing of a composition

A composition according to the first aspect of the present invention was prepared as follows:
202.5 kg of bilberry berries (36.5 wt%), 202.5 kg of lingonberry berries (36.5 wt%), and 151.3 kg of grape juice (27 wt%) were used as ingredients.

The grape juice was heated to 60-70°C (Tetra coil heat exchanger) and the berries were added to the grape juice. The mixture was cooled to under 10°C and mixed in a high shear mixer (Tetra Pak Almix 500 l). The mixture was then transferred through a big colloid mill (Fryma) and through a small colloid mill (Fryma) to a 2500 l tank to be homogenized. The mixture was then heated at 90°C for 30 seconds in conjunction with a deaerator and cooled for storing in a tank with nitrogen before being filled into 250 ml containers. The composition was in the form of a drinkable composition having a smooth mouthfeel and a consistency similar to a smoothie. The particle size of the composition was determined using a Mastersizer 2000 and a Hydro 2000SM from Malvern instruments.

The (number) volume weighted mean diameter of the particles (D[4,3]-value) was 97 µm. The (volume) volume weighted mean diameter of the particles (D[4,3]-value) was 240 µm. One serving (250 ml) of the drinkable composition had the following nutrient contents, table 1:

**Table 1: Nutrient content of drinkable composition**

| Parameter | Value | Uncertainty (±) |
|---|---|---|
| energy | 241 (kJ/100 ml) | 16.8 |
| Fat | 0.308 (g/100 ml) | 0.015 |
| Carbohydrate | 12.1 (g/100 ml) | 0.841 |
| Total sugars | 9.40 (g/100 ml) | 1.88 |
| Fiber | 1.97 (g/100 ml) | 0.393 |
| Protein | 0.582 (g/100 ml) | 0.029 |
| Salt | 0.0014 (g/100 ml) | 0.0001 |
| Water content | 91.2 (g/100 ml) | 0.912 |

The composition according to the first aspect of the present invention may have a nutrient content corresponding, within 20 % of each parameter, preferably within 10%, of each parameter, to the values given in table 1.

An analysis was made in regard of the polyphenol content of the drinkable composition, yielding the results in table 2:

**Table 2: Polyphenol content of the drinkable composition**

| Analyte | Content in mg/100g | Standard deviation |
|---|---|---|
| Delphinidin 3-0 glucoside | 25.8 | 2.31 |
| Cyanidin 3-0 glucoside | 34.8 | 3.52 |
| Pelargonidin 3-0 glucoside | 24.7 | 2.25 |
| Peonidin 3-0 glucoside | 11.4 | 0.83 |
| Malvidin 3-0 glucoside | 20.9 | 2.02 |
| Petunidin 3-0 glucoside | 19.8 | 2 |
| Cyanidin | 10.6 | 0.39 |
| Rutin | 10.2 | 1.08 |
| Quercetin-3-0-glucoside | 6.9 | 0.66 |
| Kaempferol-3-0-glucoside | 4.6 | 0.42 |
| Myricetin | 3.6 | 0.26 |
| Quercetin | 2.9 | 0.07 |
| Trans-Cinnamic acid | 1.1 | 0.04 |
| Kaempferol | 2.3 | 0.03 |
| Gallic acid | 1.7 | 0.14 |
| Chlogenic acid | 10.1 | 1.68 |
| Resveratrol | 1.2 | 0.01 |

The composition according to the first aspect of the present invention may have a polyphenol content corresponding within 20 % of each analyte, preferably within 10%, of each analyte, to the values given in table 2.

### Example 4 - Nordic berries alone improve memory and cognition in mice

Five different Nordic berries including lingonberry (*Vaccinium vitis-idaea)*, bilberry (*Vaccinium myrtillus)*, cloudberry (*Rubus chamaemorus)*, blackcurrant (*Ribes nigrum)* as well as sea buckthorn (*Hippophae rhamnoides)* were used in this study. The Nordic berries were bought as frozen whole berries and were later freeze-dried. The freeze-dried Nordic berries were then ground into fine powder using a kitchen blender. All the berry powders were flushed with nitrogen gas, vacuum-packed in bags with oxygen absorbers and stored at room temperature until being incorporated into rodent diet pellets.

All diets were formulated by Research Diets, Inc. (New Brunswick, NJ, USA) with the inclusion of essential macro- and micronutrients for rodents. A total of seven experimental diets were formulated, including low-fat (LF) control diet (10% calories from fat, modified from D12450J); high-fat (HF) [5] control diet (60% calories from fat, modified from D12492); HF diets supplemented with each of the berry powders at a dose of 6% (w/w) on dry weight basis (dwb). All HF diets were matched on macro- and micronutrients, as well as sucrose, fructose, and glucose, i.e, adjusted for the amounts coming from 6% freeze-dried berry powders added. Information about the composition of each diet is presented in Table 3. Diets were stored in bags flushed with nitrogen gas at -20 °C until use.

**Table 3. Composition of diets. All diets were designed to have an equal caloric content of fat, protein, and carbohydrates (including glucose, fructose, and sucrose).**

| | LF | HF | HF+LB | HF+BB | HF+BC | HF+CB | HF-SB |
|---|---|---|---|---|---|---|---|
| Protein | 708.0 | 708.0 | 708.0 | 708.0 | 708.0 | 708.0 | 708.0 |
| Carbohydrate | 2840.0 | 815.2 | 815.2 | 815.2 | 815.2 | 815.2 | 815.2 |
| Sucrose | 195.2 | 195.2 | 195.2 | 195.2 | 195.2 | 195.2 | 195.2 |
| Fructose | 64.0 | 64.0 | 64.0 | 64.0 | 64.0 | 64.0 | 64.0 |
| Glucose | 64.0 | 64.0 | 64.0 | 64.0 | 64.0 | 64.0 | 64.0 |
| Fat | 405.0 | 2430.0 | 2430.0 | 2430.0 | 2430.0 | 2430.0 | 2430.0 |
| Fiber | 0.0 | 0.0 | 0.0 | 0.0 | 0.0 | 0.0 | 0.0 |
| Other | 0.0 | 0.0 | 0.0 | 0.0 | 0.0 | 0.0 | 0.0 |
| Total kcals | 3953 | 3953 | 3953 | 3953 | 3953 | 3953 | 3953 |
| Calculated energy | 3.7 | 5.1 | 5.1 | 5.1 | 5.1 | 5.1 | 5.1 |
| (kcal%) | | | | | | | |
| Protein | 18 | 18 | 18 | 18 | 18 | 18 | 18 |
| Carbohydrate | 72 | 21 | 21 | 21 | 21 | 21 | 21 |
| Fat | 10 | 61 | 61 | 61 | 61 | 61 | 61 |
| Fiber | 0 | 0 | 0 | 0 | 0 | 0 | 0 |
| Total fiber¹ | 4.7 | 6.5 | 6.1 | 6.4 | 6.6 | 7.0 | 6.1 |

| | | | | | | | |
|---|---|---|---|---|---|---|---|
| ¹Fiber coming from added cellulose (LF and HF diets) or added cellulose plus fibers present in berries (berry diets) added to HF diet at 6% (w/w) dry weight basis. | | | | | | | |

One-hundred-twenty (120) male C57BI/6J mice (Janvier-Labs, Le Genest-Saint-Isle, France) arrived at the animal facility at the age of 22 weeks and were given standard chow (RM1, SDS) ad libitum for 2 weeks for acclimatization (22 °C, 12 h light-dark cycle). Subsequently, the mice were allocated into weight-matched groups (initial mean mouse weight was 29 g) of 15 animals (5 animals/cage) and randomly assigned to receiving one of the seven diets for 4 months. Animals and food were weighed, and food was replaced every week. Three mice were excluded from the analyses due to death of unknown causes before the end of the study (n = 1 from LF, n = 1 from HF, n = 1 from blackcurrant group). The study was approved by the local animal experiment ethical review committee in Lund, Sweden (approval number 5.8.18-13983/2018).

T-maze spontaneous alternation tests were conducted to assess the effects of the diets on spatial memory of the mice after 15-week feeding period. Monitoring was performed by a camera system, using the Ethovision XT 14.0 (Noldus Information Technology B.V., Wageningen, The Netherlands) software for both video tracking and video analysis. The behaviour tests were performed during the light phase.

A complete T-maze trial consisted of two phases; in the first phase the mouse was put at the starting arm for 10 seconds before the guillotine door opened, allowing it to choose between one of the goal arms. Once the mouse has chosen an arm, the guillotine door to the chosen door was shut to keep the mouse in the chosen arm for 30 seconds. For the second phase, the mouse was returned to the starting position and allowed to spontaneously choose one of the goal arms again. A total of two trials were done per mouse in a day, one in morning and the other in the afternoon. Fresh woodchip bedding was put in the maze and changed between each cage. The number of explorations in each arm was measured. First entries into correct goal arm % was evaluated as the ratio of correct arm choices (unvisited goal arm across two repeated trials) to the total number of trials, multiplied by 100.

The effects of the diets are represented in Figure 4, which illustrates improved spatial memory after 4 months consumption of the berry diets. The T-maze test revealed berry-specific differences in the ability of mice to perform in this spatial memory task. The results show that the observed first entries into correct goal arm % between maze-arms in the HF control group was not significantly different from random chance (50% first entries into correct goal arm). Mice fed LF diet showed an average first entries into correct goal arm of 71.4%, which was significantly higher (p < 0.05) compared to 50% random chance. The groups fed HF diet supplemented with bilberries (first entries into correct goal arm 83.3%, p < 0.001), blackcurrant (first entries into correct goal arm 78.6%, p < 0.01) as well as lingonberry and cloudberry (both 73.3% first entries into correct goal arm, p < 0.05) showed average first entries into correct goal arm % that are all significantly higher than a 50% random chance. No statistically significant effect was observed in the sea buckthorn group (p = 0.4985, 56.7% first entries into correct goal arm) at 4 months, however 1 month consumption of sea buckthorn resulted in improved first entries into correct goal arm % (73.3%, p = 0.0135). One sample T-test and Wilcoxon test against 50% chance was used to evaluate the results.

### FEASABLE MODIFICATIONS

The present invention is not limited to the embodiments described. This patent application is intended to cover all adjustments and variants of the preferred embodiments described herein, thus the present invention is defined by the wording of the appended claims.

It shall also be pointed out that even if it is not explicitly stated that features from a specific embodiment may be combined with features from another embodiment, the combination shall be considered obvious, if the combination is possible.

Throughout this specification and the claims which follows, unless the context requires otherwise, the word "comprise", and variations such as "comprises" or "comprising", will be understood to imply the inclusion of an entity but not the exclusion of any other entity.

## Claims

1. A composition comprising disintegrated bilberry *(Vaccinium myrtillus)* berries and disintegrated lingonberry *(Vaccinium vitis-idaea)* berries.

2. The composition according to claim 1, wherein the composition comprises at least 5 wt%, such as 5 wt% to 73 wt%, preferably 5 wt% to 67 wt%, more preferably 5 wt% to 50wt%, such as 10 wt% to 40 wt%, such as 18.25 wt% to 36.5 wt%, in total of disintegrated bilberry berries and disintegrated lingonberry berries.

3. The composition according to any preceding claim, comprising equal wt% of disintegrated bilberry berries and disintegrated lingonberry berries.

4. The composition according to any preceding claim, wherein the bilberry berries and lingonberry berries are disintegrated, such as by homogenization, such that 90% of the particles of the disintegrated berries have a diameter less than 10 µm, preferably less than 5 µm, more preferably 4.2 µm or less.

5. The composition according to any preceding claim, further comprising one or more of disintegrated cloudberry (*Rubus chamaemorus*) berries, disintegrated sea buckthorn (*Hippophaë rhamnoides)* berries, and disintegrated blackcurrant (*Ribes nigrum*) berries.

6. The composition according to any preceding claim, further comprising grape (*Vitis*) juice.

7. The composition according to any preceding claim, wherein the composition is made up essentially of disintegrated bilberry berries, disintegrated lingonberry berries, and grape juice.

8. A method of manufacturing a composition, comprising the steps of
i. providing a mixture of disintegrated bilberry berries and disintegrated lingonberry berries.

9. The method according to claim 8, further comprising the step of:
ii. mixing the composition with one or more of disintegrated cloudberry berries, disintegrated sea buckthorn berries and disintegrated blackcurrant berries.

10. The method according to any of claim 8-9, further comprising the step of:
iii. mixing the composition with grape juice.

11. The method according to any of claims 8-10, wherein homogenization is used for disintegrating.

12. A method of amelioration of a cognitive function, such as cognition, memory, and working memory, in a healthy human subject, the method comprising administering a composition according to any of the claims 1-7, to the healthy human subject.

13. The method according to claim 12, wherein the cognitive function is selected from the group consisting of spatial working memory, active working short term memory, spatial memory, working memory, memory, and cognition.

14. The method according to any of claims 12-13, wherein the composition is administered at a dose corresponding to at least 50 g, preferably at least 60 g more preferably at least 100g, of disintegrated bilberry and lingonberry berries per day.
